(19) Europäisches Patentamt
European Patent Office
Office européen des brevets

(11) Veröffentlichungsnummer: **0 024 290**
**B1**

(12) # EUROPÄISCHE PATENTSCHRIFT

(45) Veröffentlichungstag der Patentschrift: **22.12.82**

(51) Int. Cl.³: **C 07 D 473/00,** A 61 K 31/52

(21) Anmeldenummer: **80103767.2**

(22) Anmeldetag: **02.07.80**

(54) **8-Phenyl-purine, ihre Herstellung sowie sie enthaltende Arzneimittel.**

(30) Priorität: **11.07.79 DE 2927988**

(43) Veröffentlichungstag der Anmeldung: **04.03.81 Patentblatt 81/9**

(45) Bekanntmachung des Hinweises auf die Patenterteilung: **22.12.82 Patentblatt 82/51**

(84) Benannte Vertragsstaaten: **AT BE CH DE FR GB IT LI LU NL SE**

(56) Entgegenhaltungen: **Chemical Abstracts, Band 50, 1956, Columbus, Ohio, USA A. Albert, D.J. Brown «Purine Studies.I» Zusammenfassung Nr. 15540a**

(73) Patentinhaber: **Dr. Karl Thomae GmbH, Postfach 720, D-7950 Biberach (Riss) (DE)**

(72) Erfinder: **Austel, Volkhard, Dr. Dipl.-Chem, Kapellenweg 7, D-7950 Biberach 1 (DE)**
Erfinder: **Kutter, Eberhard, Dr. Dipl.Chem., Kapellenweg 12, D-7950 Biberach 1 (DE)**
Erfinder: **Heider, Joachim, Dr. Dipl.-Chem., Am Hang 3, D-7951 Warthausen 1 (DE)**
Erfinder: **Diederen, Willi, Dr., Haldenstrasse 1a, D-7950 Biberach 1 (DE)**

**EP 0 024 290 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

8-Phenyl-purine, ihre Herstellung sowie sie enthaltende Arzneimittel

Gegenstand der vorliegenden Erfindung sind neue 8-Phenyl-purine der allgemeinen Formel

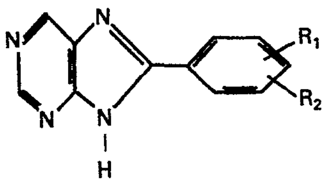

(I)

in der

$R_1$ ein Wasserstoff- oder Halogenatom, eine gegebenenfalls durch eine Alkylmercapto-, Alkylsulfinyl- oder Alkylsulfonylgruppe substituierte Alkoxygruppe, eine Alkylmercapto-, Alkylsulfinyl- oder Alkylsulfonylgruppe, wobei der Alkylteil jeweils 1 bis 3 Kohlenstoffatome enthalten kann, und

$R_2$ eine Alkoxygruppe mit 1 bis 3 Kohlenstoffatomen bedeuten, sowie deren physiologisch verträgliche Säureadditionssalze, Verfahren zu ihrer Herstellung sowie sie enthaltende Arzneimittel.

Die neuen 8-Phenyl-purine der obigen allgemeinen Formel I weisen wertvolle pharmakologische Eigenschaften auf, insbesondere positiv-inotrope Wirkungen.

Für die bei der Definition der Reste $R_1$ und $R_2$ eingangs erwähnten Bedeutungen kommt für

$R_1$ insbesondere die Bedeutung des Wasserstoff-, Fluor-, Chlor- oder Bromatoms, die der Methoxy-, Äthoxy-, Propoxy-, Isopropoxy-, Methylmercapto-, Äthylmercapto-, Isopropylmercapto-, Methylsulfinyl-, Äthylsulfinyl-, Propylsulfinyl-, Methylsulfonyl-, Äthylsulfonyl-, Propylsulfonyl-, Isopropylsulfonyl-, Methylmercaptomethoxy-, Methylsulfinylmethoxy-, Methylsulfonylmethoxy-, 2-Methylmercaptoäthoxy-, 2-Methylsulfinyläthoxy-, 2-Methylsulfonyläthoxy-, 3-Methylmercaptopropoxy-, 3-Methylsulfinylpropoxy-, 3-Methylsulfonylpropoxy-, 2-Äthylmercaptoäthoxy-, 2-Äthylsulfinyläthoxy-, 2-Äthylsulfonyläthoxy-, 3-Äthylmercaptopropoxy-, 3-Äthylsulfinylpropoxy-, 3-Äthylsulfonylpropoxy-, 2-Propylmercaptoäthoxy-, 2-Propylsulfinyläthoxy-, 2-Propylsulfonyläthoxy-, 3-Isopropylmercaptopropoxy-, 3-Isopropylsulfinylpropoxy- oder 3-Isopropylsulfonylpropoxygruppe und

für $R_2$ die der Methoxy-, Äthoxy-, Propoxy- oder Isopropoxygruppe in Betracht.

Bevorzugte Verbindungen der obigen allgemeinen Formel I sind jedoch diejenigen, in der

$R_1$ ein Wasserstoff- oder Chloratom, eine Methoxy-, Methylmercapto-, Methylsulfinyl-, Methylsulfonyl-, 2-Methylmercaptoäthoxy- oder 2-Methylsulfinyläthoxygruppe und

$R_2$ die Methoxy-, Äthoxy- oder Propoxygruppe bedeuten, wobei die Verbindungen der obigen allgemeinen Formel I besonders bevorzugt sind, in denen die Reste $R_1$ und/oder $R_2$ in 2- und/oder 4-Stellung stehen.

Erfindungsgemäss erhält man die neuen Verbindungen nach folgendem Verfahren:

Cyclisierung einer gegebenenfalls im Reaktionsgemisch hergestellten Verbindung der allgemeinen Formel

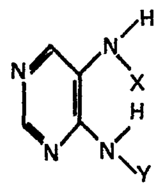

(II)

in der

einer der Reste X oder Y ein Wasserstoffatom und der andere der beiden Reste X oder Y oder beide Reste X und Y eine Gruppe der Formel

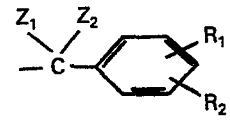

darstellen, in der

$R_1$ und $R_2$ wie eingangs definiert sind,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmässigerweise in einem Lösungsmittel wie Äthanol, Isopropanol, Eisessig, Benzol, Chlorbenzol, Toluol, Xylol, Glycol, Glycolmonomethyläther, Diäthylenglycoldimethyläther, Dimethylformamid, Tetralin oder in einem Überschuss des zur Herstellung der Verbindung der allgemeinen Formel II verwendeten Acylierungsmittels, z.B. in dem entsprechenden Nitril, Anhydrid, Säurehalogenid, Ester, Amid oder Methojodid, beispielsweise bei Temperaturen zwischen 0 und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, Sulfonylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert. butylat durchgeführt. Die Cyclisierung kann jedoch auch ohne Lösungsmittel und/oder ohne Kondensationsmittel durchgeführt werden.

Eine erfindungsgemäss erhaltene Verbindung der allgemeinen Formel I, in der $R_1$ eine Alkylmercaptogruppe enthält, kann anschliessend gewünschtenfalls mittels Oxidation in eine entsprechende Alkylsulfinylverbindung der allgemeinen Formel I übergeführt werden, und/oder eine erhaltene Verbindung der allgemeinen Formel I, in

der $R_1$ eine Alkylmercapto- oder Alkylsulfinylgruppe enthält, mittels Oxidation in eine entsprechende Alkylsulfonylverbindung der allgemeinen Formel I übergeführt werden.

Die anschliessende Oxidation wird vorzugsweise in einem Lösungsmittel, z.B. in Wasser, Wasser/Pyridin, Äthanol, Methanol, Aceton, Eisessig, Ameisensäure, verdünnter Schwefelsäure oder Trifluoressigsäure, je nach dem verwendeten Oxidationsmittel zweckmässigerweise bei Temperaturen zwischen −80 und 100 °C durchgeführt.

Zur Herstellung einer Alkylsulfinylverbindung der allgemeinen Formel I wird die Oxidation zweckmässigerweise mit einem Äquivalent des verwendeten Oxidationsmittels durchgeführt, z.B. mit Wasserstoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenzoesäure in Methylenchlorid oder Chloroform bei −20 bis 60 °C, mit Natriummetaperjodat in wässrigem Methanol oder Äthanol bei 15 bis 25 °C, mit N-Bromsuccinimid in Äthanol, mit tert.Butyl-hypochlorit in Methanol bei −80 bis −30 °C, mit Jodbenzoldichlorid in wässrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei −70 °C, der hierbei erhaltene Thioäther-Chlor-Komplex wird zweckmässigerweise mit wässrigem Äthanol hydrolysiert.

Zur Herstellung einer Alkylsulfonylverbindung der allgemeinen Formel I wird die Oxidation zweckmässigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

Ferner können die erhaltenen Verbindungen der allgemeinen Formel I gewünschtenfalls in ihre physiologisch verträglichen Salze mit starken Säuren übergeführt werden. Als Säuren kommen hierfür beispielsweise Salzsäure, Bromwasserstoffsäure, Schwefelsäure oder Phosphorsäure in Betracht.

Die als Ausgangsstoffe verwendeten Verbindungen der allgemeinen Formel II erhält man nach an sich bekannten Verfahren, z.B. durch Acylierung von 4,5-Diamino-pyrimidin.

Wie bereits eingangs erwähnt, weisen die neuen 8-Phenyl-purine der allgemeinen Formel I sowie deren physiologisch verträgliche Säureadditionssalze mit starken Säuren wertvolle pharmakologische Eigenschaften auf, insbesondere positiv inotrope Wirkungen.

Beispielsweise wurden die Verbindungen

A = 8-(2,4-Dimethoxy-phenyl)-purin,
B = 8-(2-Methoxy-4-methylmercapto-phenyl)-purin,
C = 8-[4-Methoxy-2-(2-methylmercapto-äthoxy)-phenyl]-purin,
D = 8-[4-Methoxy-2-(2-methylsulfinyl-äthoxy)-phenyl]-purin,
E = 8-(4-Methoxy-phenyl)-purin,
F = 8-(3,4-Dimethoxy-phenyl)-purin,
G = 8-(4-Chlor-2-methoxy-phenyl)-purin und
H = 8-(2-Methoxy-4-methylsulfinyl-phenyl)-purin

auf ihre biologischen Eigenschaften wie folgt untersucht:

1. Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen. Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v.

Die nachfolgende Tabelle enthält die Mittelwerte:

| Substanz | Dosis mg/kg i.v. | Blutdruckänderung mm Hg | Zunahme von $dp/dt_{max}$ % |
|---|---|---|---|
| A | 2,0 | + 42/31 | + 185 |
| B | 2,0 | + 27/17 | + 110 |
| C | 2,0 | + 33/20 | + 85 |
| D | 2,0 | + 28/10 | + 72 |
| E | 2,0 | − 8/7 | + 26 |
| F | 2,0 | − 18/23 | + 87 |
| G | 2,0 | − 0/11 | + 72 |
| H | 2,0 | − 8/13 | + 95 |

2. Akute Toxizität

Die akute Toxizität der zu untersuchenden Substanzen wurde an weissen Mäusen nach oraler Gabe einer einmaligen Dosis von 300 mg/kg orientierend bestimmt (Beobachtungszeit: 14 Tage):

| Substanz | $LD_{50}$ mg/kg p.o. |
|---|---|
| A | >300 (0 von 6 Tieren gestorben) |
| B | >300 (2 von 6 Tieren gestorben) |
| C | >300 (0 von 6 Tieren gestorben) |
| D | >300 (0 von 6 Tieren gestorben) |
| G | >300 (1 von 6 Tieren gestorben) |
| H | ~300 (3 von 6 Tieren gestorben) |

Aufgrund ihrer pharmakologischen Eigenschaften eignen sich die erfindungsgemäss hergestellten Verbindungen der allgemeinen Formel I sowie deren physiologisch verträgliche Säureadditionssalze zur Behandlung der chronischen Herzinsuffizienz und des kardiogenen Schocks.

Hierzu lassen sich die neuen 8-Phenyl-purine sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis am Erwachsenen beträgt hierbei 1–4 × täglich 25–150 mg, vorzugsweise jedoch 50–100 mg.

Die nachfolgenden Beispiele sollen die Erfindung näher erläutern:

Beispiel 1
8-(2,4-Dimethoxy-phenyl)-purin

Eine Mischung aus 2 g 4,5-Diamino-pyrimidin, 4 g 2,4-Dimethoxy-benzonitril, 6 g p-Toluolsulfonsäure-hydrat und 40 ml Benzol wird zum Sieden erhitzt und das Benzol abdestilliert. Der Rückstand wird 30 Minuten auf 120 °C erhitzt, nach dem Abkühlen mit 2n Ammoniak verrieben und mit Essigester extrahiert. Die Essigesterphase wird mit 2n Salzsäure geschüttelt, die wässrige Phase neutralisiert, wiederum mit Essigester extrahiert und diese schliesslich mit 2n Natronlauge geschüttelt. Beim Neutralisieren der wässrigen Phase fällt das Produkt aus und wird aus Wasser umkristallisiert.

Ausbeute: 0,15 g (3% der Theorie),
Schmelzpunkt: 221 °C (Zersetzung).

Beispiel 2
8-(2,4-Dimethoxy-phenyl)-purin

5,5 g 4,5-Diaminopyrimidin und 10,9 g 2,4-Dimethoxy-benzoesäure werden in einer Reibschale gut miteinander verrieben. Die Mischung wird in 100 ml Phosphoroxychlorid eingetragen und eine Stunde zum Rückfluss erhitzt. Das Phosphoroxychlorid wird durch Einrühren in Wasser zersetzt und die entstandene Lösung nach Filtration mit Ammoniak neutralisiert. Das ausgefallene Produkt wird aus Wasser und Äthanol/Cyclohexan 1:3 umkristallisiert.

Ausbeute: 3,5 g (27% der Theorie),
Schmelzpunkt: 218–220 °C.

Beispiel 3
8-(2-Methoxy-4-methylmercapto-phenyl)-purin

4,1 g 4,5-Diamino-pyrimidin-dihydrochlorid, 7,1 g 2-Methoxy-4-methylmercapto-benzoylchlorid und 50 ml Phosphoroxychlorid werden 3 Stunden zum Rückfluss erhitzt. Das Reaktionsgemisch wird auf die Hälfte eingeengt und auf Wasser gegossen. Der Niederschlag wird in siedendem Äthanol aufgelöst, durch Zusatz von Äther gefällt und durch Chromatographie an Kieselgel (Elutionsmittel: Methylenchlorid/Äthanol = 19:1) gereinigt.

Ausbeute: 2,7 g (44% der Theorie),
Schmelzpunkt: 212 °C.

Beispiel 4
8-[4-Methoxy-2-(2-methylmercapto-äthoxy)-phenyl]-purin

a) 4,5-Bis-[4-methoxy-2-(2-methylmercapto-äthoxy)-benzoylamino]-pyrimidin

Eine Mischung aus 7 g 4,5-Diamino-pyrimidin-dihydrochlorid, 26 g 4-Methoxy-2-(2-methylmercapto-äthoxy)-benzoylchlorid, 150 ml Pyridin und 2 g 4-Dimethylamino-pyridin werden 20 Stunden bei Raumtemperatur gerührt, mit Chloroform versetzt und mit Wasser gewaschen. Der Eindampfrückstand der organischen Phase wird über Kieselgel (Elutionsmittel: Methylenchlorid/Äthanol = 19:1) gereinigt.

Ausbeute: 3,3 g (15% der Theorie),
Schmelzpunkt: 139–140 °C.

b) 8-[4-Methoxy-2-(2-methylmercapto-äthoxy)-phenyl]-purin

3,1 g 4,5-Bis-[4-methoxy-2-(2-methylmercapto-äthoxy)-benzoylamino]-pyrimidin werden in 25 ml Phosphoroxychlorid 2 Stunden zum Rückfluss erhitzt. Das überschüssige Phosphoroxychlorid wird abdestilliert, der Rückstand in Wasser aufgenommen, filtriert, das Produkt durch Zugabe von Natriumbicarbonat ausgefällt und aus Äthanol umkristallisiert. Durch Reinigung der Mutterlaugen über Kieselgel (Elutionsmittel: Methylenchlorid/Äthanol = 19:1) erhält man eine weitere Fraktion.

Ausbeute: 1,43 g (81% der Theorie),
Schmelzpunkt: 173–175 °C.

Beispiel 5
8-[4-Methoxy-2-(2-methylsulfinyl-äthoxy)-phenyl]-purin

1 g 8-[4-Methoxy-2-(2-methylmercapto-äthoxy)-phenyl]-purin wird in 10 ml Eisessig gelöst und mit 0,5 ml 30%igem Wasserstoffperoxid versetzt. Nach 3,5 Stunden stehen bei Raumtemperatur verdünnt man mit Wasser, stellt mit Kaliumcarbonat alkalisch und kristallisiert den Niederschlag aus Isopropanol/Äthanol 1:1 um.

Ausbeute: 0,75 g (71% der Theorie),
Schmelzpunkt: 226–227 °C.

Beispiel 6
8-(4-Methoxy-phenyl)-purin

Hergestellt analog Beispiel 4b aus 0,35 g 4,5-Bis-(4-Methoxy-benzoylamino)-pyrimidin.

Ausbeute: 0,08 g (38% der Theorie),
Schmelzpunkt: über 300 °C.

Beispiel 7

8-(4-Chlor-2-methoxy-phenyl)-purin-hydrochlorid

a) 4-Amino-5-(4-chlor-2-methoxy-benzoyl-amino)-pyrimidin

Das aus 5,6 g 4-Chlor-2-methoxy-benzoesäure und 100 ml Thionylchlorid hergestellte 4-Chlor-2-methoxy-benzoylchlorid wird als Rohprodukt in 100 ml Pyridin gelöst und nacheinander mit 5 g 4,5-Diamino-pyrimidin-dihydrochlorid und 1 g 4-Dimethylamino-pyridin versetzt, und zunächst eine Stunde bei Raumtemperatur dann 1 1/2 Stunden bei 100 °C gerührt. Man dampft zur Trockene ein, versetzt mit Wasser und neutralisiert mit Natriumbicarbonat. Der ausgefallene Niederschlag wird ohne weitere Reinigung in der nächsten Stufe verwendet.

Ausbeute: 5,4 g (65% der Theorie),
Schmelzpunkt: 188 °C.

b) 8-(4-Chlor-2-methoxy-phenyl)-purin-hydrochlorid

3,0 g des unter a) erhaltenen Produkts werden in 50 ml Phosphoroxychlorid 4 Stunden zum Rückfluss erhitzt. Das Reaktionsgemisch wird weitgehend eingeengt und auf Wasser gegossen. Der Niederschlag wird durch Aufnehmen in Äthanol und Versetzen mit ätherischer Salzsäure in das Hydrochlorid übergeführt.

Ausbeute: 0,7 g (22% der Theorie),
Schmelzpunkt: 243–245 °C (Zersetzung, aus Äthanol).

Beispiel 8

8-(3,4-Dimethoxy-phenyl)-purin

7,5 g 4-Amino-5-(3,4-dimethoxy-benzoyl-amino)-pyrimidin werden mit 100 ml Phosphoroxychlorid 6 Stunden zum Rückfluss erhitzt, eingedampft, auf Wasser gegossen und die Lösung mit Kaliumcarbonat neutralisiert. Die Lösung wird zur Trockene eingedampft, der Rückstand mit siedendem Äthanol extrahiert. Die alkoholische Lösung wird eingedampft und das zurückbleibende Produkt über Kieselgel (Elutionsmittel: Methylenchlorid/Äthanol = 19:1) gereinigt.

Ausbeute: 1,0 g (14% der Theorie),
Schmelzpunkt: 241–243 °C.

Beispiel 9

8-(2-Methoxy-4-methylsulfinyl-phenyl)-purin

2,27 g 8-(2-Methoxy-4-methylmercapto-phenyl)-purin werden in 20 ml Trifluoressigsäure gelöst und unter Kühlung auf 5 °C 1,2 ml 30%iges Wasserstoffperoxid zugesetzt. Man rührt 3 Stunden bei Raumtemperatur und 0,5 Stunden bei 30 °C, verdünnt mit Wasser, neutralisiert mit Kaliumcarbonat und extrahiert den nach dem Eindampfen verbleibenden Rückstand mit heissem Äthanol. Das nach dem Eindampfen des Äthanols verbleibende Produkt wird über Kieselgel (Elutionsmittel: Methylenchlorid/Äthanol = 19:1) gereinigt.

Ausbeute: 1,5 g (62% der Theorie),
Schmelzpunkt: 234–236 °C.

Beispiel 10

8-(2-Methoxy-4-methylsulfonyl-phenyl)-purin

Hergestellt analog Beispiel 9 aus 2,27 g 8-(2-Methoxy-4-methylmercapto-phenyl)-purin, mit der doppelten Menge Wasserstoffperoxid.

Ausbeute: 1,1 g (45% der Theorie),
Schmelzpunkt: 244–246 °C (Zerstezung).

Beispiel 11

8-(2-Äthoxy-4-methoxy-phenyl)-purin

a) 4-Amino-5-(2-äthoxy-2-methoxy-benzoylamino)-pyrimidin

4,4 g 4,5-Diamino-pyrimidin-hydrochlorid werden in 50 ml absolutem Pyridin gelöst und dann 7,1 g 2-Äthoxy-4-methoxy-benzoylchlorid sowie 1 g 4-Dimethylamino-pyridin zugegeben. Zunächst wird 3 Stunden bei Raumtemperatur gerührt und dann weitere 3 Stunden auf 60 °C erwärmt. Das Reaktionsgemisch wird im Vakuum eingedampft, der Rückstand zweimal mit Aceton ausgekocht und schliesslich mit methanolischer Salzsäure aufgenommen und über Aktivkohle filtriert. Das Filtrat wird eingedampft, mit methanolischem Ammoniak verrührt und nach erneutem Eindampfen durch Chromatographie an Kieselgel (Elutionsmittel: Methylenchlorid/Äthanol = 100:0 bis 97:3) gereinigt.

Ausbeute: 2,3 g (27% der Theorie),
Schmelzpunkt: 170–173 °C.

b) 8-(2-Äthoxy-4-methoxy-phenyl)-purin

2,3 g 4-Amino-5-(2-äthoxy-4-methoxy-benzoylamino)-pyrimidin werden in 20 ml Phosphoroxychlorid 2,5 Stunden zum Rückfluss erhitzt. Das Reaktionsgemisch wird auf Eis gegossen und das gebildete Festprodukt abfiltriert. Das Filtrat wird mit Natriumbicarbonat alkalisch gestellt und mit Essigester extrahiert. Die Essigesterphasen werden eingedampft, der Rückstand mit dem oben gewonnenen Festprodukt vereinigt und durch Chromatographie an Kieselgel (Elutionsmittel: Methylenchlorid/Äthanol = 100:0 bis 97:3) gereinigt.

Ausbeute: 0,6 g (28% der Theorie),
Schmelzpunkt: 178–180 °C.

Beispiel 12

8-(2-n-Propyloxy-4-methoxy-phenyl)-purin-hydrochlorid

a) 4-Amino-5-(2-n-propyloxy-4-methoxy-benzoylamino)-pyrimidin

Hergestellt analog Beispiel 11a aus 4,4 g 4,5-Diaminopyrimidin-hydrochlorid und 7,6 g 2-n-Propyloxy-4-methoxy-benzoylchlorid. Das entstandene Rohprodukt wird ohne weitere Reinigung in der nächsten Stufe verwendet.

b) 8-(2-n-Propyloxy-4-methoxy-phenyl)-purin-hydrochlorid

Hergestellt analog Beispiel 11b aus 4,1 g des unter 12a erhaltenen Rohproduktes. Das Hydrochlorid wird aus Aceton mit ätherischer Salzsäure gefällt.

Ausbeute: 1,0 g (23% der Theorie), Schmelzpunkt: 221–222 °C (Zers.).

Beispiel 13

8-(2-Äthoxy-4-methylmercapto-phenyl)-purin-hydrochlorid

3,0 g 2-Äthoxy-4-methylmercapto-benzoesäure und 2,2 g 4,5-Diamino-pyrimidin-hydrochlorid werden in 30 ml Phosphoroxychlorid 3 Stunden zum Rückfluss erhitzt. Das Reaktionsgemisch wird auf Eis gegossen, ammoniakalisch gestellt und die überstehende Lösung vom halbkristallinen Rückstand abdekantiert. Dieser wird nach Auflösen in Methylenchlorid und Abfiltrieren von unlöslichen Produkten über eine Kieselgelsäule gereinigt (Elutionsmittel: Methylenchlorid/Äthanol = 100:0 bis 98:2). Das Hydrochlorid wird aus Aceton mit ätherischer Salzsäure gefällt.

Ausbeute: 1,1 g (24% der Theorie), Schmelzpunkt: 231–235 °C (Zers.).

Beispiel 14

8-(2-Äthoxy-4-methylsulfinyl-phenyl)-purin-hydrochlorid und

8-(2-Äthoxy-4-methylsulfonyl-phenyl)-purin-hydrochlorid

0,9 g 8-(2-Äthoxy-4-methylmercapto-phenyl)-purin-hydrochlorid werden bei 5 °C in 10 ml Trifluoressigsäure gelöst, mit 0,35 ml 30%igem Wasserstoffperoxid versetzt und 1 Stunde gerührt. Das Reaktionsgemisch wird mit Wasser verdünnt, mit Kaliumcarbonat alkalisch gestellt, im Vakuum eingedampft und der Rückstand mit absolutem Äthanol extrahiert. Die alkoholische Lösung wird eingeengt und an Kieselgel chromatographiert. Beim Eluieren mit Methylenchlorid/Äthanol = 100:0 bis 95:5 erhält man zuerst das Sulfon und anschliessend das Sulfoxid. Das jeweilige Hydrochlorid wird mit ätherischer Salzsäure aus Aceton gefällt.

Ausbeute: 0,15 g (15% der Theorie) 8-(2-Äthoxy-4-methylsulfinyl-phenyl)-purin-hydrochlorid vom Schmelzpunkt 173-174 °C und

0,19 g (19 % der Theorie) 8-(2-Äthoxy-4-methylsulfinyl-phenyl)-purin-hydrochlorid vom Schmelzpunkt 220–221 °C (Zers.)

Beispiel A

---

Tabletten zu 100 mg 8-(2-Methoxy-4-methylsulfinyl-phenyl)-purin

---

Zusammensetzung:

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

| | |
|---|---|
| Feuchtsiebung: | 1,5 mm |
| Trocknen: | Umlufttrockenschrank 50 °C |
| Trockensieben: | 1 mm |

Dem Granulat die restlichen Hilfsstoffe zumischen und Endmischung zu Tabletten verpressen.

| | |
|---|---|
| Tablettengewicht: | 175 mg |
| Stempel: | 8 mm ∅ |

Beispiel B

---

Dragées zu 50 mg 8-(2-Methoxy-4-methylsulfinyl-phenyl)-purin

---

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wässriger Lösung der löslichen Stärke gleichmässig befeuchten.

| | |
|---|---|
| Feuchtsiebung: | 1,0 mm |
| Trockensiebung: | 1,0 mm |
| Trocknung: | 50 °C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

Dragéegewicht: 120 mg

Beispiel C

---

Suppositorien zu 75 mg 8-(2-Methoxy-4-methylsulfinyl-phenyl)-purin

---

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

Herstellungsverfahren:

Die Zäpfchenmasse wird geschmolzen. Bei 38 °C wird die gemahlene Wirksubstanz in der Schmelze homogen dispergiert. Es wird auf 35 °C abgekühlt und in vorgekühlte Suppositorienformen ausgegossen.

Zäpfchengewicht: 1,7 g

Beispiel D

---

Ampullen zu 50 mg 8-(2-Methoxy-4-methylsulfinyl-phenyl)-purin

---

1 Ampulle enthält:

| | | |
|---|---|---|
| Wirksubstanz | | 50,0 mg |
| Sorbit | | 250,0 mg |
| Dest. Wasser | ad | 5,0 ml |

Herstellungsverfahren:

Die Wirksubstanz und Sorbit werden in dest. Wasser gelöst, dann wird auf das angegebene Volumen aufgefüllt und steril filtriert.

Abfüllung: in Ampullen zu 5 ml
Sterilisation: 20 Minuten bei 120 °C

Beispiel E

Tropfen zu 250 mg pro 5 ml 8-(2-Methoxy-4-methylsulfinyl-phenyl)-purin

| | | |
|---|---|---|
| Wirksubstanz | 5,0 | g |
| p-Oxybenzoesäuremethylester | 0,035 | g |
| p-Oxybenzoesäurepropylester | 0,015 | g |
| Anisöl | 0,05 | g |
| Menthol | 0,06 | g |
| Saccharin-Natrium | 1,0 | g |
| Glycerin | 10,0 | g |
| Äthanol | 40,0 | g |
| Dest. Wasser ad | 100,0 | ml |

Herstellungsverfahren:

Die Benzoesäureester werden in Äthanol gelöst und anschliessend das Anisöl und das Menthol zugegeben. Dann wird die Wirksubstanz, Glycerin und Saccharin-Natrium im Wasser gelöst zugegeben. Die Lösung wird anschliessend klar filtriert.

**Patentansprüche für die Vertragsstaaten: BE, CH, DE, FR, GB, IT, LI, LU, NL, SE.**

1. 8-Phenyl-purine der allgemeinen Formel

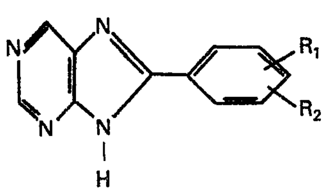

(I)

in der

$R_1$ ein Wasserstoff- oder Halogenatom eine gegebenenfalls durch eine Alkylmercapto-, Alkylsulfinyl- oder Alkylsulfonylgruppe substituierte Alkoxygruppe, eine Alkylmercapto-, Alkylsulfinyl- oder Alkylsulfonylgruppe, wobei der Alkylteil jeweils 1 bis 3 Kohlenstoffatome enthalten kann, und

$R_2$ eine Alkoxygruppe mit 1 bis 3 Kohlenstoffatomen bedeuten, und deren physiologisch verträgliche Säureadditionssalze mit Säuren.

2. Verbindungen der allgemeinen Formel I gemäss Anspruch 1, in der

$R_1$ ein Wasserstoff- oder Chloratom, eine Methoxy-, Methylmercapto-, Methylsulfinyl-, Methylsulfonyl-, 2-Methylmercaptoäthoxy- oder 2-Methylsulfinyläthoxygruppe und

$R_2$ die Methoxy-, Äthoxy- oder Propoxygruppe bedeuten, und deren physiologisch verträgliche Säureadditionssalze mit Säuren.

3. Verbindungen der allgemeinen Formel I gemäss Anspruch 1, in der

$R_1$ und $R_2$ wie im Anspruch 2 definiert sind und die Reste $R_1$ und/oder $R_2$ in 2- und/oder 4-Stellung des Phenylkerns stehen, und deren physiologisch verträgliche Säureadditionssalze mit Säuren.

4. 8-(2,4-Dimethoxy-phenyl)-purin und dessen Säureadditionssalze.

5. 8-(2-Methoxy-4-methylmercapto-phenyl)-purin und dessen Säureadditionssalze.

6. 8-(2-Methoxy-4-methylsulfinyl-phenyl)-purin und dessen Säureadditionssalze.

7. 8-[4-Methoxy-2-(2-methylsulfinyl-äthoxy)-phenyl]-purin und dessen Säureadditionssalze.

8. Arzneimittel, enthaltend eine Verbindung gemäss den Ansprüchen 1–7 neben einem oder mehreren inerten Trägerstoffen und/oder Verdünnungsmitteln.

9. Arzneimittel gemäss Anspruch 8 geeignet zur Behandlung der chronischen Herzinsuffizienz und des kardiogenen Schocks.

10. Verfahren zur Herstellung von neuen 8-Phenyl-purinen der allgemeinen Formel

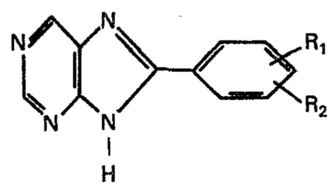

(I)

in der

$R_1$ ein Wasserstoff- oder Halogenatom, eine gegebenenfalls durch eine Alkylmercapto-, Alkylsulfinyl- oder Alkylsulfonylgruppe substituierte Alkoxygruppe, eine Alkylmercapto-, Alkylsulfinyl- oder Alkylsulfonylgruppe, wobei der Alkylteil jeweils 1 bis 3 Kohlenstoffatome enthalten kann, und

$R_2$ eine Alkoxygruppe mit 1 bis 3 Kohlenstoffatomen bedeuten, sowie von deren physiologisch verträglichen Salzen mit starken Säuren, dadurch gekennzeichnet, dass eine gegebenenfalls im Reaktionsgemisch hergestellte Verbindung der allgemeinen Formel

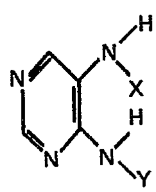

(II)

in der

einer der Reste X oder Y ein Wasserstoffatom und der andere der beiden Reste X oder Y oder beide Reste X und Y eine Gruppe der Formel

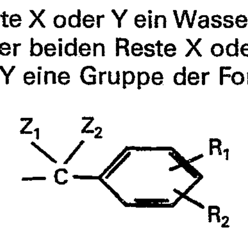

darstellen, in der

$R_1$ und $R_2$ wie eingangs definiert sind,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen der

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird

und gewünschtenfalls anschliessend eine erhaltene Verbindung der allgemeinen Formel I, in der $R_1$ eine Alkylmercaptogruppe enthält, mittels Oxidation in eine entsprechende Alkylsulfinylverbindung der allgemeinen Formel I übergeführt wird und/oder eine erhaltene Verbindung der allgemeinen Formel I, in der $R_1$ eine Alkylmercapto- oder Alkylsulfinylgruppe enthält, mittels Oxidation in eine entsprechende Alkylsulfinylverbindung übergeführt wird und/oder

eine erhaltene Verbindung der allgemeinen Formel I in ihre physiologisch verträglichen Säureadditionssalze mit starken Säuren übergeführt wird.

**Patentansprüche für den Vertragsstaat: AT**

1. Verfahren zur Herstellung von neuen 8-Phenyl-purinen der allgemeinen Formel

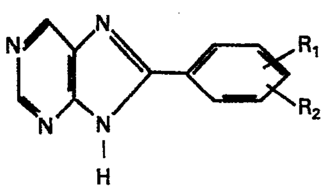

(I)

in der

$R_1$ ein Wasserstoff- oder Halogenatom, eine gegebenenfalls durch eine Alkylmercapto-, Alkylsulfinyl- oder Alkylsulfonylgruppe substituierte Alkoxygruppe, eine Alkylmercapto-, Alkylsulfinyl- oder Alkylsulfonylgruppe, wobei der Alkylteil jeweils 1 bis 3 Kohlenstoffatome enthalten kann, und

$R_2$ eine Alkoxygruppe mit 1 bis 3 Kohlenstoffatomen bedeuten, sowie von deren physiologisch verträglichen Salzen mit starken Säuren, dadurch gekennzeichnet, dass eine gegebenenfalls im Reaktionsgemisch hergestellte Verbindung der allgemeinen Formel

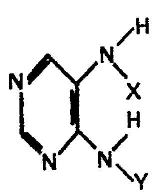

(II)

in der

einer der Reste X oder Y ein Wasserstoffatom und der andere der beiden Reste X oder Y oder beide Reste X und Y eine Gruppe der Formel

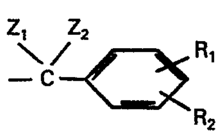

darstellen, in der

$R_1$ und $R_2$ wie eingangs definiert sind,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder $Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird

und gewünschtenfalls anschliessend eine erhaltene Verbindung der allgemeinen Formel I, in der $R_1$ eine Alkylmercaptogruppe enthält, mittels Oxidation in eine entsprechende Alkylsulfinylverbindung der allgemeinen Formel I übergeführt wird und/oder eine erhaltene Verbindung der allgemeinen Formel I, in der $R_1$ eine Alkylmercapto-oder Alkylsulfinylgruppe enthält, mittels Oxidation in eine entsprechende Alkylsulfonylverbindung übergeführt wird und/oder

eine erhaltene Verbindung der allgemeinen Formel I in ihre physiologisch verträglichen Säureadditionssalze mit starken Säuren übergeführt wird.

2. Verfahren gemäss Anspruch 1, dadurch gekennzeichnet, dass die Umsetzung in einem Lösungsmittel durchgeführt wird.

3. Verfahren gemäss den Ansprüchen 1 und 2, dadurch gekennzeichnet, dass die Cyclisierung bei Temperaturen zwischen 0 und 250 °C durchgeführt wird.

4. Verfahren gemäss den Ansprüchen 1–3, dadurch gekennzeichnet, dass die Cyclisierung bei der Siedetemperatur des Reaktionsgemisches durchgeführt wird.

5. Verfahren gemäss den Ansprüchen 1–4, dadurch gekennzeichnet, dass die Cyclisierung in Gegenwart eines Kondensationsmittels oder einer Base durchgeführt wird.

**Revendications pour les Etats contractants: BE, CH, DE, FR, GB, IT, LI, LU, NL, SE**

1. 8-phényl-purines de formule générale

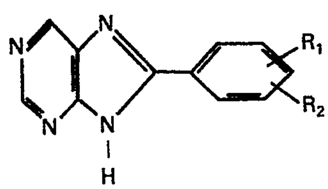

(I)

dans laquelle

$R_1$ représente un atome d'hydrogène ou d'halogène, un groupe alcoxy éventuellement substitué par un groupe alcoylmercapto, alcoylsulfinyle ou alcoylsulfonyle, ou représente un groupe alcoylmercapto, alcoylsulfinyle ou alcoylsulfonyle, la partie alcoyle pouvant dans chaque cas contenir 1 à 3 atomes de carbone, et

$R_2$ représente un groupe alcoxy avec 1 à 3 atomes de carbone, et leurs sels d'addition physiologiquement supportables avec des acides.

2. Composés de formule générale I selon la revendication 1, dans laquelle

$R_1$ représente un atome d'hydrogène ou de chlore, un groupe méthoxy, méthylmercapto, méthylsulfinyle, méthylsulfonyle, 2-méthylmercaptoéthoxy ou 2-méthylsulfinyléthoxy, et

$R_2$ représente le groupe méthoxy, éthoxy ou propoxy, et leurs sels d'addition physiologiquement supportables avec des acides.

3. Composés de formule générale I selon la revendication 1, dans laquelle

$R_1$ et $R_2$ sont définis comme dans la revendication 2 et les radicaux $R_1$ et/ou $R_2$ sont en position 2 et/ou 4 du noyeau phényle, et leurs sels d'addition physiologiquement supportables avec des acides.

4. La 8-(2,4-diméthoxy-phényl)-purine et ses sels d'addition d'acides.

5. La 8-(2-méthoxy-4-méthylmercapto-phényl)-purine et ses sels d'addition d'acides.

6. La 8-(2-méthoxy-4-méthylsulfinyl-phényl)-purine et ses sels d'addition d'acides.

7. La 8-[4-méthoxy-2-(2-méthylsulfinyl-éthoxy)-phényl]-purine et ses sels d'addition d'acides.

8. Médicament contenant un composé selon les revendication 1–7 conjointement à un ou plusieurs excipients et/ou diluants inertes.

9. Médicament selon la revendication 8 adapté au traitement des insuffisances cardiaques chroniques et du chock cardiogène.

10. Procédé pour la préparation des nouvelles 8-phényl-purines de formule générale

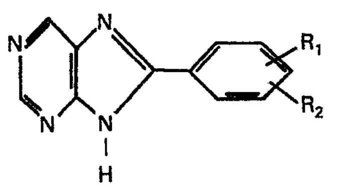

(I)

dans laquelle

$R_1$ représente un atome d'hydrogène ou d'halogène, un groupe alcoxy éventuellement substitué par un groupe alcoylmercapto, alcoylsulfinyle ou alcoylsulfonyle, ou représente un groupe alcoylmercapto, alcoylsulfinyle ou alcoylsulfonyle, la partie alcoyle pouvant dans chaque cas contenir 1 à 3 atomes de carbone, et

$R_2$ représente un groupe alcoxy avec 1 à 3 atomes de carbone, ainsi que de leurs sels physiologiquement supportables avec des acides forts, caractérisé en ce que l'on cyclise un composé éventuellement préparé dans le milieu réactionnel de formule générale

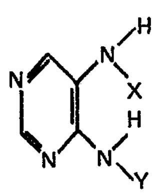

(II)

dans laquelle

l'un des radicaux X ou Y représente un atome d'hydrogène et l'autre des deux radicaux X ou Y ou les deux radicaux X et Y représentent un groupe de formule

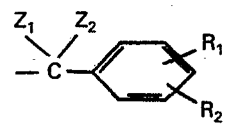

dans laquelle

$R_1$ et $R_2$ sont définis comme au début,

$Z_1$ et $Z_2$, qui peuvent être identiques ou différents, représentent des groupes amino éventuellement substitués ou des groupes hydroxy ou mercapto éventuellement substitués par des groupes alcoyle inférieur ou $Z_1$ et $Z_2$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone, et en ce qu'éventuellement on transforme ensuite un composé obtenue de formule générale I dans laquelle $R_1$ contient un groupe alcoylmercapto, au moyen d'une oxydation en un composé alcoylsulfinylé correspondant de formule générale I et/ou on transforme un composé obtenue de formule générale I dans laquelle $R_1$ contient un groupe alcoylmercapto ou alcoylsulfinyle, au moyen d'une oxydation en un composé alcoylsulfonylé correspondant et/ou

on transforme un composé obtenu de formule générale I en ses sels physiologiquement supportables d'addition avec des acides forts.

**Revendications pour l'Etat contractant: AT**

1. Procédé pour la préparation des nouvelles 8-phényl-purines de formule générale

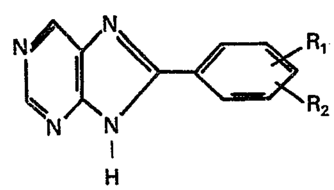

(I)

dans laquelle

$R_1$ représente un atome d'hydrogène ou d'halogène, un groupe alcoxy éventuellement substitué par un groupe alcoylmercapto, alcoylsulfinyle ou alcoylsulfonyle, ou représente un groupe alcoylmercapto, alcoylsulfinyle ou alcoylsulfonyle, la partie alcoyle pouvant dans chaque cas contenir 1 à 3 atomes de carbone, et

$R_2$ représente un groupe alcoxy avec 1 à 3 atomes de carbone, ainsi que leurs sels physiologiquement supportables avec des acides forts, caractérisé en ce qu'on cyclise un composé éventuellement préparé dans le mélange réactionnel de formule générale

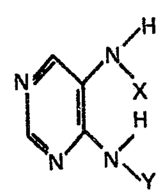

(II)

dans laquelle l'un des radicaux X ou Y représente un atome d'hydrogène et l'autre des deux radicaux X ou Y ou les deux radicaux X et Y représentent un groupe de formule

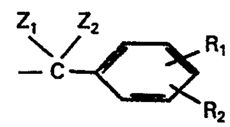

dans laquelle

$R_1$ et $R_2$ sont définis comme au début,

$Z_1$ et $Z_2$, qui peuvent être identiques ou différents, représentent des groupes amino éventuellement substitués ou des groupes hydroxy ou mercapto éventuellement substitués par des groupes alcoyle inférieur ou $Z_1$ et $Z_2$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone,

et en ce qu'éventuellement on transforme ensuite un composé obtenu de formyle génerale I dans laquelle $R_1$ contient un groupe alcoylmercapto, au moyen d'une oxydation en un composé alcoylsulfinylé correspondant de formule générale I et/ou on transforme un composé obtenu de formule générale I dans laquelle $R_1$ contient un groupe alcoylmercapto ou alcoylsulfinyle au moyen d'une oxydation en un composé alcoylsulfonylé correspondant et/ou

on transforme un composé obtenu de formule générale I en ses sels physiologiquement supportables d'addition avec des acides forts.

2. Procédé selon la revendication 1, caractérisé en ce que la réaction est effectuée dans un solvant.

3. Procédé selon les revendications 1 et 2, caractérisé en ce que la cyclisation est effectuée à des températures entre 0 et 250 °C.

4. Procédé selon les revendications 1–3, caractérisé en ce que la cyclisation est effectuée à la température d'ébullition du mélange réactionnel.

5. Procédé selon les revendications 1–4, caractérisé en ce que la cyclisation est effectuée en présence d'un agent de condensation ou d'une base.

**Claims for the contracting states:**
**BE, CH, DE, FR, GB, IT, LI, LU, NL, SE**

1. 8-Phenyl-purines of general formula

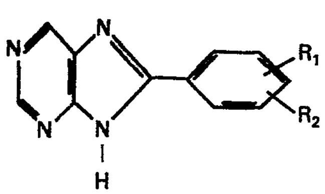

(I)

wherein

$R_1$ represents a hydrogen or halogen atom; an alkoxy group optionally substituted by an alkylmercapto, alkylsulphinyl or alkylsulphonyl group; or an alkylmercapto, alkylsulphinyl or alkylsulphonyl group, wherein the alkyl part may contain 1 to 3 carbon atoms, and

$R_2$ represents an alkoxy group with 1 to 3 carbon atoms,

and the physiologically acceptable acid addition salts thereof.

2. Compounds of general formula I as claimed in claim 1, wherein

$R_1$ represents a hydrogen or chlorine atom, a methoxy, methylmercapto, methylsulphinyl, methylsulphonyl, 2-methylmercaptoethoxy or 2-methylsulphinylethoxy group and

$R_2$ represents the methoxy, ethoxy or propoxy group, and the physiologically acceptable acid addition salts thereof.

3. Compounds of general formula I as claimed in claim 1, wherein

$R_1$ and $R_2$ are as defined in claim 2 and the groups $R_1$ and/or $R_2$ are in the 2- and/or 4-position of the phenyl nucleus,

and the physiologically acceptable acid addition salts thereof.

4. 8-(2,4-Dimethoxy-phenyl)-purine and the acid addition salts thereof.

5. 8-(2-Methoxy-4-methylmercapto-phenyl)-purine and the acid addition salts thereof.

6. 8-(2-Methoxy-4-methylsulphinyl-phenyl)-purine and the acid addition salts thereof.

7. 8-[4-Methoxy-2-(2-methylsulphinyl-ethoxy)-phenyl]-purine and the acid addition salts thereof.

8. Pharmaceutical compositions containing a compound as claimed in claims 1 to 7 together with one or more inert carriers and/or diluents.

9. Pharmaceutical composition as claimed in claim 8, suitable for the treatment of chronic cardiac insufficiency and cardiogenic shock.

10. Process for the preparation of new 8-phenylpurines of general formula

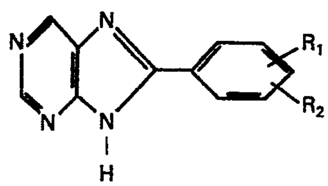

(I)

wherein

$R_1$ represents a hydrogen or halogen atom; an alkoxy group optionally substituted by an alkylmercapto, alkylsulphinyl or alkylsulphonyl group; or

an alkylmercapto, alkylsulphinyl or alkylsulphonyl group, wherein the alkyl part may contain 1 to 3 carbon atoms and

$R_2$ represents an alkoxy group with 1 to 3 carbon atoms, and of the physiologically acceptable salts thereof with strong acids, characterised in that a compound of general formula

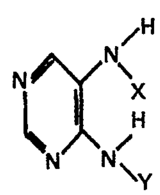

(II)

optionally prepared in the reaction mixture, wherein

one of the groups X or Y represents a hydrogen atom and the other group X or Y or both groups X and Y represent a group of formula

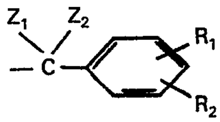

wherein

$R_1$ and $R_2$ are as hereinbefore defined,

$Z_1$ and $Z_2$, which may be identical or different, represent optionally substituted amino groups or hydroxy or mercapto groups optionally substituted by lower alkyl groups, or $Z_1$ and $Z_2$ together represent an oxygen or sulphur atom, an imino group optionally substituted by an alkyl group with 1 to 3 carbon atoms, or an alkylenedioxy or alkylenedithio group each having 2 or 3 carbon atoms, is cyclised

and if desired a compound of general formula I thus obtained wherein $R_1$ contains an alkylmercapto group is subsequently converted by oxidation into a corresponding alkylsulphinyl compound of general formula I, and/or

a compound of general formula I obtained, wherein $R_1$ contains an alkylmercapto or alkylsulphinyl group, is converted by oxidation into a corresponding alkylsulphonyl compound, and/or

a compound of general formula I obtained is converted into the physiologically acceptable acid addition salts thereof with strong acids.

**Claims for the Contracting state: AT**

1. Process for the preparation of new 8-phenylpurines of general formula

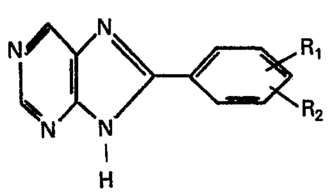

(I)

wherein

$R_1$ represents a hydrogen or halogen atom; an alkoxy group optionally substituted by an alkylmercapto, alkylsulphinyl or alkylsulphonyl group; or

an alkylmercapto, alkylsulphinyl or alkylsulphonyl group, wherein the alkyl part may contain 1 to 3 carbon atoms, and

$R_2$ represents an alkoxy group with 1 to 3 carbon atoms, and of the physiologically acceptable salts thereof with strong acids, characterised in that a compound of general formula

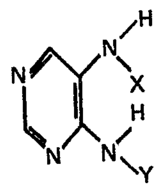

(II)

optionally prepared in the reaction mixture, wherein

one of the groups X or Y represents a hydrogen atom and the other group X or Y or both groups X and Y represent a group of formula

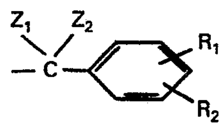

wherein

$R_1$ and $R_2$ are as hereinbefore defined,

$Z_1$ and $Z_2$, which may be identical or different, represent optionally substituted amino groups or hydroxy or mercapto groups optionally substituted by lower alkyl groups, or $Z_1$ and $Z_2$ together represent an oxygen or sulphur atom, an imino group optionally substituted by an alkyl group with 1 to 3 carbon atoms, or an alkylenedioxy or alkylendithio group each having 2 or 3 carbon atoms, is cyclised

and if desired a compound of general formula I thus obtained wherein $R_1$ contains an alkylmercapto group is subsequently converted by oxidation into a corresponding alkylsulphinyl compound of general formula I, and/or a compound of general formula I obtained, wherein $R_1$ contains an alkylmercapto or alkylsulphinyl group, is converted by oxidation into a corresponding alkylsulphonyl compound, and/or

a compound of general formula I obtained is converted in the physiologically acceptable acid addition salts thereof with strong acids.

2. Process as claimed in claim 1, characterised in that the reaction is effected in a solvent.

3. Process as claimed in claims 1 and 2, characterised in that the cyclisation is effected at temperatures of between 0 and 250 °C.

4. Process as claimed in claims 1 to 3, characterised in that the cyclisation is effected at the boiling temperature of the reaction mixture.

5. Process as claimed in claims 1 to 4, characterised in that the cyclisation is effected in the presence of a condensing agent or a base.